## Europäisches Patentamt

## European Patent Office

## Office européen des brevets

(19)

(11) Veröffentlichungsnummer: **0 190 617**
**B1**

(12)

# EUROPÄISCHE PATENTSCHRIFT

(45) Veröffentlichungstag der Patentschrift:
**01.06.88**

(51) Int. Cl.⁴: **B 01 J 23/72** // (C07C29/136, 31:12)

(21) Anmeldenummer: **86100890.2**

(22) Anmeldetag: **23.01.86**

(54) Verfahren zur Herstellung eines Kupfer und Siliciumoxid enthaltenden Katalysators.

(30) Priorität: **02.02.85 DE 3503587**

(43) Veröffentlichungstag der Anmeldung:
**13.08.86 Patentblatt 86/33**

(45) Bekanntmachung des Hinweises auf die Patenterteilung:
**01.06.88 Patentblatt 88/22**

(84) Benannte Vertragsstaaten:
**BE DE FR GB IT**

(56) Entgegenhaltungen:
**EP - A - 0 020 048**
**EP - A - 0 064 241**
**DE - A - 2 726 710**

(73) Patentinhaber: **BASF Aktiengesellschaft, Carl-Bosch-Strasse 38, D-6700 Ludwigshafen (DE)**

(72) Erfinder: **Broecker, Franz Josef, Dr., Schwanthaler Allee 20, D-6700 Ludwigshafen (DE)**
Erfinder: **Fischer, Rolf, Dr., Bergstrasse 98, D-6900 Heidelberg (DE)**
Erfinder: **Malsch, Klaus-Dieter, Amselweg 14, D-6707 Schifferstadt (DE)**
Erfinder: **Reiss, Wolfgang, Dr., Bannwasserstrasse 70, D-6700 Ludwigshafen (DE)**
Erfinder: **Schnabel, Rolf, Dr., Frankenstrasse 22, D-6707 Schifferstadt (DE)**
Erfinder: **Weitz, Hans-Martin, Dr., Auf dem Koeppel 40, D-6702 Bad Duerkheim (DE)**

## Beschreibung

Die Erfindung betrifft ein Verfahren zur Herstellung eines Kupfer und Siliciumoxid enthaltenden Katalysators und die Verwendung des so hergestellten Katalysators für die Hydrierung von Dicarbonsäuredialkylestern zu Diolen.

Kupfer und Siliciumoxid enthaltende Katalysatoren und ihre Eignung für die Hydrierung von Ketonen, Aldehyden und Carbonsäureestern sind bereits bekannt. So wird z.B. in der US-A-3 756 964 ein Katalysator beschrieben, den man durch Tränken eines anorganischen Oxids, wie Silicalgel mit Lösungen von Kupfersalzen und Chromsäure und stufenweises Erhitzen herstellt. Ein für die Hydrierung von Oxalsäurediester zu Glykol geeigneter Kupfer-Katalysator wird in der EP-A-64 241 beschrieben. Dieser Katalysator wird durch Zusammengeben einer Aminkomplexverbindung des Kupfers mit kolloidalem Silicagel hergestellt. In der japanischen Patentveröffentlichung 45 337/1972 wird ein Verfahren zur Herstellung von γ-Butyrolacton beschrieben, bei dem man die Ester der entsprechenden Dicarbonsäuren in Gegenwart eines Katalysators hydriert, der zu 10 bis 70 Gew.-% aus Kupfer, zu 5 bis 40 Gew.-% aus einem Erdalkalifluorid und zu 20 bis 80 Gew.-% aus Diatomeenerde besteht.

Die DE-A-2 726 710 beschreibt die Herstellung eines Cu/SiO₂-Katalysators und dessen Verwendung zur Hydrierung von Dicarbonsäuremthylestern. Der Katalysator wird hergestellt durch Vermischen einer Kupfernitratlösung mit einer Wasserglaslösung, die überschüssiges Kaliumhydroxid enthält, wobei während des Vermischens ein pH-Wert von 7 in dem Reaktionsgemisch eingehalten wird.

Da diese bekannten Katalysatoren bei der Hydrierung von Dicarbonsäuredialkylestern zu den entsprechenden Diolen nur eine unzureichende Aktivität und zu geringe Selektivität aufweisen, bestand die Aufgabe, einen hochaktiven und selektriven Katalysator herzustellen, mit dem die Hydrierung der Carbonsäureester zur Diolherstellung auch bei einer kontinuierlichen Arbeitsweise wirtschaftlich betrieben werden kann.

Es wurde nun gefunden, dass man einen Kupfer und Siliciumoxid enthaltenden Katalysator, mit dem sich die angestrebten Vorteile erreichen lassen, erhält, wenn man eine wässrige Alkalisilikatlösung mit einem SiO₂-Gehalt von 1,0 bis 26 Gew.-% mit Salpetersäure auf einen pH-Wert von 0,5 bis 2,0 stellt, nach der Salpetersäurezugabe eine wässrige Kupfernitratlösung mit einem Cu-Gehalt von 5 bis 15 Gew.-% so zugibt, dass eine klare Lösung entsteht, die pro Mol SiO₂ 0,18 bis 3,0 Mol Cu enthält, die erhaltene Lösung bei Temperaturen von 40 bis 80°C mit einer wässrigen Alkalicarbonatlösung vermischt, wobei man im Gemisch einen pH-Wert von 7,5 bis 8,5 einhält, den dabei ausfallenden Niederschlag abtrennt, trocknet, durch Erhitzen auf 250 bis 400°C calziniert und auf an sich übliche Weise zu Katalysatorformlingen verpresst.

Als wässrige Alkalisilikatlösung verwendet man zweckmässigerweise Wasserglas, das man mit Wasser auf den gewünschten SiO₂-Gehalt verdünnt. Die Salpetersäure wird z.B. als 20- bis 60%- ige Säure zugegeben. Danach wird die Kupfernitratlösung zugegeben. Die Herstellung dieser Ausgangslösung erfolgt bei Temperaturen von 20 bis 40, vorzugsweise bei Raumtemperatur. Wichtig ist, dass die salpetersaure Silikatlösung nach Zugabe der Kupfernitratlösung noch klar ist.

Man vermischt die Kupfer enthaltende Silikatlösung mit der wässrigen Alkalicarbonatlösung vorteilhaft so, dass man die beiden Lösungen unter Rühren zusammengibt, zweckmässigerweise durch gleichmässige und gleichzeitige Eingabe in eine Vorlage von Wasser, wobei man den Zulauf so reguliert, dass während des ganzen Fällungsvorgangs ein pH-Wert von 7,5 bis 8,5 eingehalten wird. Die Temperatur im Gemisch hält man dabei z.B. auf 40 bis 80°C. Als wässrige Alkalicarbonatlösung verwendet man z.B. eine 5- bis 25gewichtsprozentige Natriumcarbonat- oder Kaliumcarbonatlösung.

Der beim Vermischen ausgefallene Niederschlag wird abgetrennt und zweckmässigerweise mit Wasser nitratfrei gewaschen. Dann wird er auf an sich übliche Weise getrocknet, durch Erhitzen auf 250 bis 400°C, vorzugsweise 300 bis 350°C calziniert und zu Katalysatorformlingen verpresst. Beispielsweise stellt man die Formlinge nach Zugabe von 0,5 bis 2,5 Gew.-% Graphit durch Verpressen her, wobei die Druckfestigkeit der Formlinge 350 bis 450 Kp/cm² (357 bis 459 bar) betragen sollte. Es hat sich auch als vorteilhaft erwiesen, die Formlinge nach dem Verpressen z.B. 2 bis 5 Stunden einer Nachcalzinierung bei Temperaturen von 250 bis 350 zu unterziehen.

Die nach dem erfindungsgemässen Verfahren erhältlichen Katalysatoren eignen sich hervorragend für die Hyrierung von Dicarbonsäuredialkylestern zu Diolen. Als Dicarbonsäuredialkylester kommen z.B. Dialkylester von C₄-C₆-Dicarbonsäuren, wie Bernsteinsäure, Glutarsäure und Adipinsäure in Betracht, wobei die Alkylreste z.B. 1 bis 12 C-Atome enthalten können. Bei der Hydrierung der genannten Ester zur Herstellung der entsprechenden Diole übertreffen die nach dieser Erfindung zu verwendenden Katalysatoren die bekannten Katalysatoren durch eine höhere Raum-Zeit-Ausbeute und Selektivität.

Man hydriert die Dicarbonsäuredialkylester auf an sich bekannte Weise in einem Reaktor bei Temperaturen von 80 bis 200°C und Drücken von 1 bis 400 bar, vorzugsweise 50 bis 300 bar. Dabei geht man praktisch so vor, dass man den Katalysator in den Reaktor einbaut und zunächst durch Überleiten eines Gemisches aus Stickstoff und Wasserstoff reduziert. Diese Reduktion des Katalysators erfolgt z.B. bei Temperaturen zwischen 100 und 200°C. Die Wasserstoffmenge wird dabei so dosiert, dass eine Maximaltemperatur von 230°C nicht überschritten wird. Mit dem auf diese Weise aktivierten Katalysator kann dann die Hydrierung des Esters erfolgen.

### Beispiel 1

Zur Katalysatorherstellung stellt man separat die folgenden zwei Lösungen her:

*Lösung a)* 2,328 kg Wasserglas mit einem SiO₂-Gehalt von 24,8 Gew.-% werden in 36 l Wasser gelöst. Der pH-Wert der erhaltenen wässrigen Lösung wird unter guter Rührung durch Zugabe von 30%iger Salpetersäure auf pH = 2,0 eingestellt.

Dann gibt man 13,307 kg einer wässrigen Kupfernitratlösung mit einem Kupfergehalt von 14,4 Gew.-% bei Raumtemparatur hinzu. Die Lösung muss nach der Zugabe von Kupfernitrat noch klar sein.

*Lösung b)* Diese Lösung wird durch Auflösen von 5,12 kg Natriumcarbonat in 16 l Wasser hergestellt.

In einem beheizbaren Rührkessen werden 10 l Wasser von 60°C vorgelegt. Dann werden innerhalb von 60 Minuten Lösung (a) und Lösung (b) parallel und gleichzeitig in den Rührkessen gepumpt. Die Temparatur wird dabei auf 60°C gehalten und die Zulaufmenge so gesteuert, dass während der ganzen Fällungszeit im Reaktionsgemisch ein pH-Wert von 8,0 eingehalten wird. Nach Beendigung der Fällung wird noch 30 Minuten nachgerührt und der Niederschlag abfiltriert. Der Filterkuchen wird mit Wasser nitratfrei gewaschen und dann bei 110°C getrocknet. Man erhält 3,8 kg trockenes Produkt mit einem Glühverlust (2 h bei 900°C) von 27 Gew.-%. Nach dem Trocknen wird 7 Stunden bei 300°C calziniert. Man erhält 2,9 kg calziniertes Produkt der Zusammensetzung 60% Cu und 18,4% $SiO_2$ mit einem Glühverlust von 5,6% (2 h bei 900°C). Nach dem Zerkleinern des Produktes auf eine Korngrösse von < 1,5 mm wird 1 Gew.-% Graphit zugemischt. Dann wird der Katalysator zu 5 × 5-mm-Pillen verpresst.

Die Druckfestigkeit der Pillen sollte 350 bis 450 $kp/cm^2$ (357 - 459 bar) betragen. Nach dem Verpressen werden die Pillen nochmals 4 Stunden bei 300°C nachcalziniert. Man erhält etwa 2,9 kg Pillen mit einem Schüttgewicht von etwa 1,0 kg/l.

### Beispiel 2

Der nach Beispiel 1 hergestellte Katalysator wird wie folgt verwendet:

In einem 1,5-l-Rührautoklaven werden 94 g des in Beispiel 1 dargestellten Katalysators eingebaut und unter Stickstoff auf 100°C aufgeheizt. Anschliessend wird dem Stickstoff 1 Vol.-% Wasserstoff zugegeben. Unter Durchleiten dieser Gasmischung aus 99 Vol.-% $N_2$ und 1 Vol.-% $H_2$ wird die Temparatur stündlich um 20°C gesteigert bis auf 180°C. Bei dieser Temparatur wird der Katalysator nach 12 Stunden durch weiteres Durchleiten des Gemisches aus $H_2$ und $N_2$ reduziert und dann mit Stickstoff gespült. Nun wird der Autoklav mit 900 g Bernsteinsäuredibutylester beschickt und der Ester unter folgenden Reaktionsbedingungen hydriert:

| | |
|---|---|
| Temparatur: | 180°C |
| $H_2$-Druck: | 280 bar |
| Turbinendrehzahl: | 2000 U/min. |

Nach 10 bzw. 20 Stunden wurden dem Autoklaven Flüssigkeitsproben entnommen und mittels Gaschromatographie analysiert. Es ergaben sich folgende Konzentrationen im Austrag:

| | | nach 10 Std. | nach 20 Std. |
|---|---|---|---|
| Bernsteinsäuredibutylester Gew.-% | | 23 | 3 |
| Butanol | » | 48 | 63 |
| Butandiol | » | 21 | 33 |
| Tetrahydrofuran | » | 0,1 | 0,3 |

### Beispiel 3 (Vergleichsbeispiel)

Beispiel 2 wurde wiederholt, wobei jedoch als Katalysator 100 ml (= 139 g) des im Handel unter der Bezeichnung «Girdler 9-13» erhältlichen Katalysators (Form: 3,5 × 4,5-mm-Tabletten, Zusammensetzung 38,6% Cu, 26,5% Cr, 0,4% Mn) verwendet wurde. Die entsprechenden Konzentrationen im Auftrag waren:

| | | nach 10 Std. | nach 20 Std. |
|---|---|---|---|
| Bernsteinsäuredibutylester Gew.-% | | 75 | 66 |
| Butanol | » | 14 | 24 |
| Butandiol | » | 4 | 8 |
| Tetrahydrofuran | » | 0,6 | 1,1 |

Der in diesem Beispiel verwendete Katalysator und seine Anwendung bei der Hydrierung von aliphatischen Estern wird z.B. in Charles L. Thomas: «Catalytic Processes and proven Catalysts» Academic Press, New York and London, 1970, beschrieben.

### Beispiel 4 (Vergleichsbeispiel)

Beispiel 2 wurde wiederholt, wobei jedoch als Katalysator 100 ml des im Handel unter der Bezeichnung R3-11 (BASF) erhältlichen Katalysators (From: 4,5 × 5,0-mm-Tabletten, Zusammensetzung 40% Cu auf Magnesiumsilikat) verwendet wurde. Die entsprechenden Konzentrationen im Austrag betrugen:

| | | nach 10 Std. | nach 20 Std. |
|---|---|---|---|
| Bernsteinsäuredibutylester Gew.-% | | 50 | 31 |
| Butanol | » | 27 | 39 |
| Butandiol | » | 10 | 14 |
| Tetrahydrofuran | » | 0,3 | 0,6 |

Die nach den Beispielen 2 bis 4 erzielten Resultate sind in der folgenden Tabelle zusammengefasst:

*Raum-Zeit-Ausbeute (RAZ)* (g Butandiol pro 1 Katalysatorvolumen und Std.) und

*Katalysator-Aktivität* (g Butandiol pro kg Katalysator und Std.) für die ersten 10 Std. Versuchsdauer:

*Budantiol/Tetrahydrofuran (THF)-Verhältnis* nach 20 Std. Versuchsdauer

| Beispiel-Nr. | RZA (g/l·h) | Kat.-Aktivität (g/kg·h) | Butandiol: THF |
|---|---|---|---|
| 2 | 189 | 239 | 110 |
| 3 | 36 | 26 | 7 |
| 4 | 90 | 115 | 23 |

### Beispiel 5

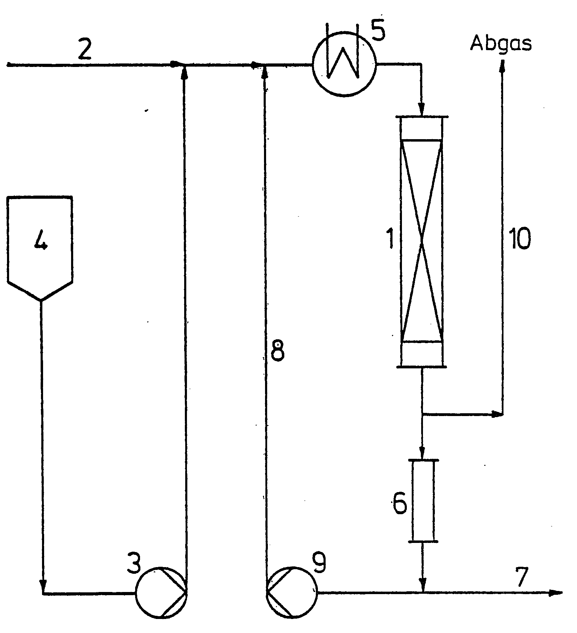

51 l des nach Beispiel 1 hergestellten Katalysators wurden in den Reaktor (1) einer durch die Figur schematisch dargestellten Kreislaufapparatur eingebaut. Die Apparatur wurde unter Zuleitung von Stickstoff (2) auf 100°C aufgeheizt. Dann wurde über die Zuleitung (2) ein Gemisch aus Stickstoff und Wasserstoff (1 bis 2 Vol.-% $H_2$) zur Reduzierung des Kataly-

sators durch die Apparatur geleitet. Man reduzierte 60 h bei 200°C. Nach dieser Katalysatorreduktion wurde der zu hydrierende Bernsteinsäuredibutylester mit der Zulaufpumpe (3) aus dem Behälter (4) zusammen mit dem Wasserstoff an der Zuleitung (2) in den Hydrierkreislauf gepumpt. Mit Hilfe des Wärmeaustauschers (5) wurde die Temparatur auf 190°C eingestellt. Über die Standhaltung im Abscheider (6) wurde eine dem Zulauf entsprechende Produktmenge kontinuierlich ausgeschleust (7). Die Produktrückführung (8) erfolgte mit der Kreislaufpumpe (9). Das Abgas wurde über die Ableitung (10) abgeleitet. Die Hydrierung des Bernsteinsäuredibutylesters zu Butandiol wurde unter folgenden Reaktionsbedingungen durchgeführt:

| | |
|---|---|
| $H_2$-Partialdruck: | 250 bar |
| Temparatur: | 190°C |
| Abgasmenge: | 1,3 ($m^3$(h) |
| Flüssigkeitskreislauf: | 350 (l/h) |
| Querschnittsbelastung: | 35 ($m^3/m^2 \cdot h$) |

Es wurden folgende Ergebnisse erzielt:

| | nach 10 Std. | nach 20 Std. |
|---|---|---|
| Raum-Zeit-Ausbeute RZA $\left(\dfrac{\text{kg Butandiol}}{1 \text{ Kat.} \cdot \text{h}}\right)$ | 0,17 | 0,24 |
| Umsatz (%) | 92 | 87 |
| Selektivität (%) | 99 | 99 |

*Beispiel 6* (Vergleichsbeispiel)

Es wurde wie in Beispiel 5 beschrieben verfahren, wobei jedoch als Katalysator der in Beispiel 4 genannte Katalysator verwendet wurde. Dabei wurden die folgenden Ergebnisse erhalten:

| | nach 10 Std. | nach 20 Std. |
|---|---|---|
| RZA $\left(\dfrac{\text{kg Butandiol}}{1 \text{ Kat.} \cdot \text{h}}\right)$ | 0,11 | 0,12 |
| Umsatz (%) | 72 | 48 |
| Selektivität (%) | 97 | 94 |

Die beiden letzten Beispiele zeigen, dass der erfindungsgemäss verwendete Katalysator dem bekannten Kupferkatalysator R3-11 hinsichtlich Raum-Zeit-Ausbeute, Umsatz und Selektivität erheblich überlegen ist.

## Patentansprüche

1. Verfahren zur Herstellung eines Kupfer und Siliciumoxid enthaltenden Katalysators, dadurch gekennzeichnet, dass man eine wässrige Alkalisilikatlösung mit einem $SiO_2$-Gehalt von 1 bis 26 Gew.-% mit Salpetersäure auf einen pH-Wert von 0,5 bis 2,0 stellt, nach der Salpetersäurezugabe eine wässrige Kupfernitratlösung mit einem Cu-Gehalt von 5 bis 15 Gew.-% so zugibt, dass eine klare Lösung entsteht, die pro Mol $SiO_2$ 0,18 bis 3,0 Mol Cu enthält, die erhaltene Lösung bei Temperaturen von 40 bis 80°C mit einer wässrigen Alkalicarbonatlösung vermischt, wobei man im Gemisch einen pH-Wert von 7,5 bis 8,5 einhält, den dabei ausfallenden Niederschlag abtrennt, trocknet, durch Erhitzen auf 250 bis 400°C calziniert und auf an sich übliche Weise zu Katalysatorformlingen verpresst.

2. Verwendung des nach Anspruch 1 erhaltenen Katalysators für die Hydrierung von Dicarbonsäuredialkylestern zu Diolen.

3. Verwendung des nach Anspruch 1 erhaltenen Katalysators für die Hydrierung von Bernsteinsäuredibutylester zu Butandiol.

## Claims

1. A process for the preparation of a catalyst containing copper and silica, wherein an aqueous alkali metal silicate solution containing from 1 to 26% by weight of $SiO_2$ is brought to a pH of from 0.5 to 2.0 with nitric acid, an aqueous copper nitrate solution containing from 5 to 15% by weight of Cu is added, after the addition of the nitric acid, so that a clear solution containing from 0.18 to 3.0 moles of Cu per mole of $SiO_2$ is formed, the resulting solution is mixed with an aqueous alkali metal carbonate solution at from 40 to 80°C, the pH of the mixture being maintained at from 7.5 to 8.5, and the precipitated material is separated off, dried, calcined by heating at from 250 to 400°C and pressed in a conventional manner to give catalyst moldings.

2. The use of a catalyst obtained according to claim 1 for the hydrogenation of dialkyl dicarboxylates to diols.

3. The use of a catalyst obtained according to claim 1 for the hydrogenation of dibutyl succinate to butanediol.

## Revendications

1. Procédé de préparation d'un catalyseur contenant du cuivre et de l'oxyde de silicium, caractérisé en ce que l'on ajuste le pH d'une solution aqueuse de silicate d'un métal alcalin avec une teneur en $SiO_2$ comprise entre 1 et 26% en poids, avec de l'acide nitrique, entre 0,5 et 2,0 et, après l'addition de l'acide nitrique, on ajoute une solution aqueuse de nitrate de cuivre, d'une teneur en cuivre de 5 à 15% en poids, de façon telle que l'on obtient une solution limpide avec 0,18 à 3,0 moles de Cu par mole de $SiO_2$, à laquelle on mélange, entre 40 et 80°C et en maintenant le pH entre 7,5 et 8,5, une solution aqueuse de carbonate d'un métal alcalin, on sépare le précipité formé, qui est séché et calciné par un chauffage entre 250 et 400°C, puis comprimé de manière usuelle en éléments de catalyseur façonnés.

2. Utilisation d'un catalyseur préparé (par le procédé) suivant la revendication 1 pour l'hydrogénation d'esters dialkyliques d'acides dicarboxyliques en diols.

3. Utilisation d'un catalyseur préparé (par le procédé) suivant la revendication 1 pour l'hydrogénation de l'ester dibutylique de l'acide succinique en butanediol.